# EUROPEAN PATENT APPLICATION

(11) **EP 3 243 508 A1**
(43) Date of publication of application: **15.11.2017**
(21) Application number: 17153541.2
(22) Date of filing: 05.09.2013
(51) Int. Cl.: A61K 9/28, A61K 31/4412

(54) **COATED PHARMACEUTICAL COMPOSITION CONTAINING REGORAFENIB**

(30) Priority: 06.09.2012 EP 12183331
(62) Divisional of application: 13762967.1
(71) Applicant: Bayer Healthcare LLC, Whippany, NJ 07981 (US)
(72) Inventor: SKRABS, Susanne, 50859 Köln (DE); FUNKE, Adrian, 14055 Berlin (DE); KRESSE, Mayk, 14195 Berlin (DE); OBERDIECK, Ulrich, 14165 Berlin (DE)
(74) Representative: BIP Patents

(57) **Abstract**

The present invention relates to a coated pharmaceutical composition containing regorafenib, a hydrate, solvate, metabolite or pharmaceutically acceptable salt thereof or a polymorph thereof and its process of preparation and its use for treating disorders.

## Description

The present invention relates to a coated pharmaceutical composition containing regorafenib, a hydrate, solvate, metabolite or pharmaceutically acceptable salt thereof or a polymorph thereof and its process of preparation and its use for treating disorders.

Regorafenib which is 4{4-[3-(4-chloro-3-trifluoromethylphenyl)-ureido]-3-fluorophenoxy}-pyridine-2-carboxylic acid methylamide, a compound of formula (I) 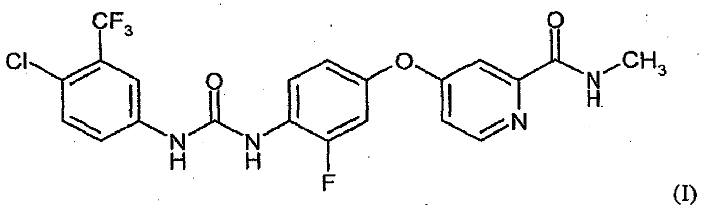 is a potent anti-cancer and anti-angiogenic agent that possesses various activities including inhibitory activity on the VEGFR, PDGFR, raf, p38, and/or flt-3 kinase signalling molecules and it can be used in treating various diseases and conditions like hyper-proliferative disorders such as cancers, tumors, lymphomas, sarcomas and leukemias as described in WO 2005/009961. Furthermore salts of the compound of formula (I) such as its hydrochloride, mesylate and phenylsulfonate are mentioned in WO 2005/009961. The monohydrate of the compound of formula (I) is mentioned in WO 2008/043446. An improved process for the manufacturing of regorafenib in high purity is described in WO 2011/128261. Due to the limited solubility of regorafenib monohydrate (see table 1) an applicable pharmaceutical composition containing regorafenib is in form of a solid dispersion as described in WO 2006/026500.

**Table 1: thermodynamic solubility of regorafenib monohydrate in different solvents**

| **Solvent.** | **Solubility (mg/ml)** |
|---|---|
| Water | <0.1 |
| Ligth liquid paraffin | <0.1 |
| Ethanol | 6.4 |
| Polyethylenglycol (PEG) 400 | 67.3 |
| HPB-Cyclodextrin/water (10:90) | < 0.1 |
| PEG 400/water (30:70) | 0.27 |
| Oleoylpolyethylenglycol glycerides | 3.6 |

The preferred route of drug administration is through the oral cavity. This route provides the greatest comfort and convenience of dosing. Tablets are preferred forms of pharmaceutical compositions for oral administration. In order to administrate solid formulations conveniently a coating is often needed. Objective of a coating can be to provide a homogeneous appearance, to mask discoloration during storage, to add color for product identification, to mask a bad taste, to prevent dusting during handling, to prevent abrasion or friction of a tablet, to increase mechanical stability, to facilitate and give a more convenient feeling when swallowing the tablet, in particular when the dimensions are large, to provide light protection for the drug or to protect the drug against humidity. Typical tablet coating agents are hydroxyethyl cellulose, hydroxypropyl cellulose, methyl cellulose, hydroxypropyl methyl cellulose, sucrose, liquid glucose, ethyl cellulose, cellulose acetate phthalate and shellac. The coating agents can be mixed with further applicable coating excipients or commercially available ready-to-use coating mixtures can be used like Opadry™ II 85G35294 pink, Opadry™ II 85G25457 red, Opadry™ II 85G23665 orange. The coating temperature usually depends on the type of coating agent and solvent used. Polyvinyl alcohol based coatings are typically processed at bed temperatures of 45-48°C (inlet air temperature 60-65°C). Often even higher temperatures are used for other coating materials. When using aqueous solvents in the coating procedure (e.g. the outlet air temperature) coating is usually conducted at higher temperatures.

The problem to be solved by the present invention is to provide a coated pharmaceutical composition containing regorafenib in high purity, in particular directly after the coating and/or after storage.

Surprisingly the pharmaceutical composition according to the invention shows a reduced degradation of the active agent.

The present invention pertains to a pharmaceutical composition comprising regorafenib which is the compound of the formula (I) 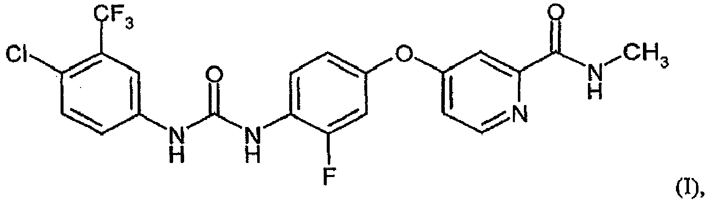 a hydrate, solvate, metabolite or pharmaceutically acceptable salt of regorafenib, or a polymorph thereof and at least one pharmaceutically acceptable excipient wherein the pharmaceutical composition is coated by a coating comprising a polyvinyl alcohol based polymer and optionally one or more further pharmaceutically acceptable excipients.

The pharmaceutical compositions according to the present invention can be utilized to achieve the desired pharmacological effect by administration to a patient in need thereof. A patient, for the purpose of this invention, is a mammal, including a human, in need of treatment for the particular condition or disease. Therefore, the present invention includes pharmaceutical compositions which are comprised of a pharmaceutically acceptable excipient and a pharmaceutically effective amount of a compound of the invention. A pharmaceutically acceptable excipient is any excipient which is relatively non-toxic and innocuous to a patient at concentrations consistent with effective activity of the active ingredient so that any side effects ascribable to the carrier do not vitiate the beneficial effects of the active ingredient. A pharmaceutically effective amount of compound is that amount which produces a result or exerts an influence on the particular condition being treated.

The term "the compound of formula (I)" or "regorafenib" refer to 4-{4-[({[4-chloro-3-(trifluoromethyl)phenyl]amino}carbonyl)amino]-3-fluorophenoxy}-*N-*methylpyridine-2-carboxamide as depicted in formula (I).

The term "compound of the invention" or "active agent" or "active ingredient" refer to regorafenib, a hydrate, solvate, metabolite or pharmaceutically acceptable salt of regorafenib, or a polymorph thereof.

Solvates for the purposes of the invention are those forms of the compounds or their salts where solvent molecules form a stoichiometric complex in the solid state and include, but are not limited to for example water, ethanol and methanol.

Hydrates are a specific form of solvates, where the solvent molecule is water. Hydrates of the compounds of the invention or their salts are stoichiometric compositions of the compounds or salts with water, such as, for example, hemi-, mono- or dihydrates. Preference is given to the monohydrate of regorafenib.

Salts for the purposes of the present invention are preferably pharmaceutically acceptable salts of the compounds according to the invention. Suitable pharmaceutically acceptable salts are well known to those skilled in the art and include salts of inorganic and organic acids, such as hydrochloric acid, hydrobromic acid, sulfuric acid, phosphoric acid, methanesulphonic acid, trifluoromethanesulfonic acid, benzenesulfonic acid, *p*-toluenesulfonic acid (tosylate salt), 1-naphthalenesulfonic acid, 2-naphthalenesulfonic acid, acetic acid, trifluoroacetic acid, malic acid, tartaric acid, citric acid, lactic acid, oxalic acid, succinic acid, fumaric acid, maleic acid, benzoic acid, salicylic acid, phenylacetic acid, and mandelic acid. In addition, pharmaceutically acceptable salts include salts of inorganic bases, such as salts containing alkaline cations (e.g., Li⁺ Na⁺ or K⁺), alkaline earth cations (e.g., Mg⁺² , Ca⁺² or Ba⁺²), the ammonium cation, as well as acid salts of organic bases, including aliphatic and aromatic substituted ammonium, and quaternary ammonium cations, such as those arising from protonation or peralkylation of triethylamine, *N,N-*diethylamine, *N,N-*dicyclohexylamine, lysine, pyridine, *N,N-*dimethylaminopyridine (DMAP), 1,4-diazabiclo[2.2.2]octane (DABCO), 1,5-diazabicyclo[4.3.0]non-5-ene (DBN) and 1,8-diazabicyclo[5.4.0]undec-7-ene (DBU). Preference is given to the hydrochloride, mesylate or phenylsulfonate salt of regorafenib.

Metabolites of regorafenib for the purpose of the present invention include 4-[4-({[4-chloro-3-(trifluoromethyl)phenyl]carbamoyl}amino)-3-fluorophenoxy]-N-methylpyridine-2-carboxamide 1-oxide, 4-[4-({[4-chloro-3-(trifluaromethyl)phenyl]carbamoyl}amino)-3-fluorophenoxy]-N-(hydroxymethyl)pyridine-2-carboxamide, 4-[4-({[4-chloro-3-(trifluoromethyl)phenyl]carbamoyl]amino)-3-fluorophenoxy]pyridine-2-carboxamide and 4-[4-({[4-chloro-3-(trifluoromethyl)phenyl]carbamoyl}amino)-3-fluorophenoxy]pyridine-2-carboxamide 1-oxide.

Preferred are regorafenib and the monohydrate of regorafenib as a compound of the present invention.

The total amount of the active ingredient (compound of the invention) to be administered preferably via the oral route using the pharmaceutical composition of the present invention will generally range from about 0.1 mg/kg to about 50 mg/kg body weight per day. Based upon standard laboratory techniques known to evaluate compounds useful for the treatment of hyper-proliferative disorders, by standard toxicity tests and by standard pharmacological assays for the determination of treatment of the conditions identified above in mammals, and by comparison of these results with the results of known medicaments that are used to treat these conditions, the effective dosage of the pharmaceutical compositions of this invention can readily be determined by those skilled in the art. The amount of the administered active ingredient can vary widely according to such considerations as the particular compound and dosage unit employed, the mode and time of administration, the period of treatment, the age, sex, and general condition of the patient treated, the nature and extent of the condition treated, the rate of drug metabolism and excretion, the potential drug combinations and drug-drug interactions, and the like.

Preference is given to an amount of the compound of the invention in the pharmaceutical composition from 4 to 400 mg, preferably from 10 to 200 mg, more preferably from 10 to 100 mg.

An aspect of the invention of particular interest is a pharmaceutical composition comprising regorafenib in an amount of 4 to 400 mg, preferably from 10 to 200 mg, more preferably from 10 to 100 mg.

The daily dose of the compound of the present invention, in particular regorafenib, is from 10 to 1000 mg, preferably 40 to 500 mg, more preferably 80 to 320 mg, e.g. 160 mg.

The pharmaceutical composition according to the invention is administered one or more, preferably up to three, more preferably up to two times per day. Preference is given to an administration via the oral route.

Nevertheless, it may in some cases be advantageous to deviate from the amounts specified, depending on body weight, individual behavior toward the active ingredient, type of preparation and time or interval over which the administration is affected. For instance, less than the aforementioned minimum amounts may be sufficient in some cases, while the upper limit specified has to be exceeded in other cases. In the case of administration of relatively large amounts, it may be advisable to divide these into several individual doses over the day.

This pharmaceutical composition will be utilized to achieve the desired pharmacological effect by preferably oral administration to a patient in need thereof, and will have advantageous properties in terms of drug release, bioavailability, and/or compliance in mammals. A patient, for the purpose of this invention, is a mammal, including a human, in need of treatment for the particular condition or disease.

Preference is given to a pharmaceutical composition which is a an immediate release tablet.

The pharmaceutical composition according to the invention is preferably a solid pharmaceutical compositions and is administered orally or rectally, preferably orally.

The pharmaceutical composition of the present invention includes any solid formulation which is applicable to be coated.

Pharmaceutical compositions according to the invention include but are not limited to granules, pellets, tablets, dragées, pills, melts or solid dispersions and may be prepared according to methods known to the art for the manufacture of pharmaceutical compositions. Preference is given to tablets, solid dispersions, pellets and granules. Most preferably the pharmaceutically compositions according to the invention is a tablet.

An aspect of the invention of particular interest is a pharmaceutical composition in the form of a solid dispersion or a pharmaceutical composition comprising a solid dispersion. The solid dispersion may be a solid solution, glass solution, glass suspension, amorphous precipitation in a crystalline carrier, eutectic or monotectic, compound or complex formation or combinations thereof.

A solid dispersion according to the present invention comprises at least a compound of the invention and a pharmaceutically acceptable matrix.

The term "matrix" or "matrix agents" as used herein refers to both polymeric excipients, non-polymeric excipients and combinations thereof, capable of dissolving or dispersing the compound of the invention.

An aspect of the invention of particular interest is a pharmaceutical composition comprising a solid dispersion, wherein the matrix comprises a pharmaceutically acceptable polymer, such as polyvinylpyrrolidone, vinylpyrrolidone/vinylacetate copolymer, polyalkylene glycol (i.e. polyethylene glycol), hydroxyalkyl cellulose (i.e. hydroxypropyl cellulose), hydroxyalkyl methyl cellulose (i.e. hydroxypropyl methyl cellulose), carboxymethyl cellulose, sodium carboxymethyl cellulose, ethyl cellulose, polymethacrylates, polyvinyl alcohol, polyvinyl acetate, vinyl alcohol/vinyl acetate copolymer, polyglycolized glycerides, xanthan gum, carrageenan, chitosan, chitin, polydextrin, dextrin, starch, proteins or a mixture thereof.

Another aspect of the invention is a pharmaceutical composition comprising a solid dispersion, wherein the matrix comprises a sugar and/or sugar alcohol and/or cyclodextrin, for example sucrose, lactose, fructose, maltose, raffinose, sorbitol, lactitol, mannitol, maltitol, erythritol, inositol, trehalose, isomalt, inulin, maltodextrin, ß-cyclodextrin, hydroxypropyl-ß-cyclodextrin or sulfobutyl ether cyclodextrin or a mixture thereof.

In a preferred embodiment at least one from the group of polyvinylpyrrolidone, copovidone, hydroxypropyl cellulose, hydroxypropyl methyl cellulose, polyethylene glycol and polyethylene oxide is used as matrix agent in the solid dispersion. More preferably polyvinylpyrrolidone and/or hydroxypropyl cellulose are used as matrix agents. Most preferably polyvinylpyrrolidone is used as matrix agent.

An embodiment of particular interest the solid dispersion comprises the compound of the invention (calculated as solvent-free regorafenib base which is the compound of formula (I)) and the matrix agent in a weight ratio of 1:0.5 to 1:20, preferably 1:1 to 1:10, most preferably 1:1 to 1:5.

Additional suitable excipients that are useful in the formation of the matrix of the solid dispersion include, but are not limited to alcohols, organic acids, organic bases, amino acids, phospholipids, waxes, salts, fatty acid esters, polyoxyethylene sorbitan fatty acid esters, and urea.

The solid dispersion may contain certain additional pharmaceutical acceptable ingredients, such as surfactants, fillers, disintegrants, recrystallization inhibitors, plasticizers, defoamers, antioxidants, detackifier, pH-modifiers, glidants and lubricants.

Another aspect of the invention of particular interest are solid dispersions containing croscarmellose sodium, sodium starch glycolate, crospovidone, low substituted hydroxypropyl cellulose (L-HPC), starch, microcrystalline cellulose or a combination thereof as carrier or disintegrant. Preferably the solid dispersion comprises microcrystalline cellulose and/or croscarmellose sodium.

In another preferred embodiment, the solid dispersion comprises polyvinylpyrrolidone, croscarmellose sodium and optionally microcrystalline cellulose.

An embodiment of particular interest the solid dispersion comprises the compound of the invention (calculated as solvent-free regorafenib base which is the compound of formula (I)) and the sum of carrier and disintegrant in a weight ratio of 1:0.5 to 1:20, preferably 1:1 to 1:10, most preferably 1:1 to 1:6.

The solid dispersion of the invention can be prepared according to methods known to the art for the manufacture of solid dispersions, such as fusion/melt technology, hot melt extrusion, solvent evaporation (i.e. freeze drying, spray drying or layering of powders of granules), coprecipitation, supercritical fluid technology and electrostatic spinning method which are for example described in WO 2006/026500.

Hot melt extrusion or solvent evaporation techniques are preferred processes for preparation of solid dispersion formulations of this invention.

A solvent suitable for manufacture of solid dispersions by solvent evaporation processes such as spray-drying, layering or fluid-bed granulation can be any compound, wherein the compound of the invention can be dissolved. Preferred solvents include alcohols (e.g. methanol, ethanol, n-propanol, isopropanol, and butanol), ketones (e.g. acetone, methyl ethyl ketone and methyl isobutyl ketone), esters (e.g. ethyl acetate and propyl acetate) and various other solvents such as acetonitrile, methylene chloride, chloroform, hexane, toluene, tetrahydrofurane, cyclic ethers, and 1,1,1-trichloroethane. Lower volatility solvents, such as dimethyl acetamide or dimethyl sulfoxide can also be used. Mixtures of solvents, such as 20% ethanol and 80% acetone, can also be used, as can mixtures with water as long as the drug and if necessary the matrix agent are sufficiently soluble to make the process practicable.

In a preferred embodiment the solvent used for manufacture of the solid dispersion is methanol, ethanol, n-propanol, isopropanol, acetone or a mixture thereof. More preferably a mixture of ethanol and acetone is used as solvent.

An aspect of the invention of particular interest is a composition, wherein the solid dispersion is substantially homogeneous.

An aspect of the invention of particular interest is a pharmaceutical composition, in which the compound of the invention is substantially amorphous.

The coating of the pharmaceutical composition of the present invention comprises a polyvinyl alcohol based polymer as film-forming agent. The polyvinyl alcohol based polymer according to the present invention includes but is not limited to fully hydrolysed polyvinyl alcohol polymer, partially hydrolysed polyvinyl alcohol polymer (contains free alcohol groups and esterified alcohol groups i.e. as acetate) esterified polyvinyl alcohol polymer for example polyvinyl acetate polymer, a co-polymer of the aforementioned with polyethylene glycol for example a polyvinyl alcohol-polyethylene glycol co-polymer or a mixture of the aforementioned. Preference is given to a partially hydrolysed polyvinyl alcohol polymer.

The polyvinyl alcohol based polymer in the coating is present in an amount of 30 to 70%, preferably 35 to 60%, more preferably 35 to 50% by weight of the total coating.

Furthermore the coating of the pharmaceutical composition of the present invention comprises optionally one or more further pharmaceutically acceptable excipients such as plasticizers, colorants, opacifiers, anti-tacking agents, dispersing agents and suspending agents.

Plasticizers which may be used in the coating include but are not limited to polyethylene glycol, propylene glycol, sorbitol, glycerol, maltitol, xylitol, mannitol, erythritol, glycerol trioleate, tributyl citrate, triethyl citrate acetyl triethyl citrate, glyceryl triacetate, stearic acid, medium chain triglycerides or a mixture thereof. Preference is given to polyethylene glycol, medium chain triglycerides and/or stearic acid.

The plasticizer in the coating may be present in an amount of 5 to 30%, preferably 8 to 25%, more preferably 10 to 20% by weight of the total coating.

Colorants which may be used in the coating include but are not limited to ferric oxide red, ferric oxide yellow, ferric oxide black, titanium dioxide, indigotine, sunset yellow FCF, tartrazin, erythrosine, quinoline yellow, carbon black, anthocyanin, riboflavin, carmine, curcumin, chlorophyll, carotene or a mixture thereof. Preference is given to ferric oxides and titanium dioxide.

The colorants in sum in the coating are present in an amount of 5 to 40%, preferably 8 to 30%, more preferably 10 to 20% by weight of the total coating.

Anti-tacking agents which may be used in the coating include but are not limited to talc, magnesium stearate, stearic acid, lecithin, soy lecithin, mineral oil, carnauba wax, acetylated monoglycerides, polysorbate or a mixture thereof. Preference is given to talc, lecithin, soy lecithin, and polysorbate.

Anti-tacking agents in sum in the coating are present in an amount of 3 to 30%, preferably 5 to 25%, more preferably 10 to 20% by weight of the total coating.

Opacifiers which may be used in the coating include by are not limited to talc and titanium dioxide. Opacifiers in sum in the coating are present in an amount of 10 to 45%, preferably 15 to 35%, more preferably 15 to 25% by weight of the total coating.

The coating material can be prepared from the individual components as mentioned before. Alternatively ready-to-use mixtures can be used which include but are not limited to for example Opadry™ II 85G35294 pink, Opadry™ II 85G25457 red, Opadry™ II 85G23665 orange (provided by Colorcon), Kollicoat™ IR white (provided by BASF), Sepifilm™ IR (provided by SEPPIC). Preference is given to Opadry™ II 85G35294 pink, Opadry™ II 85G25457, red, Opadry™ II 85G23665 orange.

An aspect of the invention of particular interest is a pharmaceutical composition which is a tablet comprising regorafenib, a hydrate, solvate, metabolite or pharmaceutically acceptable salt of regorafenib, or a polymorph thereof, preferably regorafenib, and at least one pharmaceutically acceptable excipient wherein the pharmaceutical composition is coated by a coating comprising a polyvinyl alcohol based polymer and optionally one or more further pharmaceutically acceptable excipients.

An aspect of the invention of particular interest is a pharmaceutical composition comprising a solid dispersion comprising regorafenib and at least one pharmaceutically acceptable excipient wherein the pharmaceutical composition is coated by a coating comprising a polyvinyl alcohol based polymer and optionally one or more further pharmaceutically acceptable excipients.

Preference is given to a pharmaceutical composition which is a tablet comprising a solid dispersion comprising regorafenib and at least one pharmaceutically acceptable excipient wherein the pharmaceutical composition is coated by a coating comprising a polyvinyl alcohol based polymer and optionally one or more further pharmaceutically acceptable excipients.

More preferably the pharmaceutical composition according to the present invention is a tablet comprising a solid dispersion comprising regorafenib and at least a pharmaceutically acceptable matrix agent selected from the group consisting of polyvinylpyrrolidone, vinylpyrrolidone/vinylacetate copolymer, polyalkylene glycol like polyethylene glycol, hydroxyalkyl cellulose like hydroxypropyl cellulose, hydroxyalkyl methyl cellulose like hydroxypropyl methyl cellulose, carboxymethyl cellulose, sodium carboxymethyl cellulose, ethyl cellulose, polymethacrylates, polyvinyl alcohol, polyvinyl acetate, vinyl alcohol/vinyl acetate copolymer, polyglycolized glycerides, xanthan gum, carrageenan, chitosan, chitin, polydextrin, dextrin, starch, proteins or a mixture thereof, preferably polyvinylpyrrolidone, wherein the pharmaceutical composition is coated by a coating comprising a polyvinyl alcohol based polymer and optionally one or more further pharmaceutically acceptable excipients.

Most preferably the pharmaceutical composition according to the present invention is a tablet comprising a solid dispersion comprising regorafenib, polyvinylpyrrolidone as pharmaceutically acceptable matrix agent and microcrystalline cellulose and/or croscarmellose sodium as further pharmaceutically acceptable excipients wherein the pharmaceutical composition is coated by a coating comprising a polyvinyl alcohol based polymer in particular a partially hydrolysed polyvinyl alcohol polymer and optionally one or more further pharmaceutically acceptable excipients.

In this connection the pharmaceutical composition according to the invention - when investigated for release testing - contains 4-(4-amino-3-fluorophenoxy)pyridine-2-carboxylic acid methylamide (IUPAC: 4-(4-amino-3-fluorophenoxy)-N-methylpyridine-2-carboxamide) (AFP-PMA) in an amount of equal or less than 0.050%, that means from 0.001% to a maximum of 0.050%, preferably in an amount of equal or less than 0.025%, that means from 0.001% to a maximum of 0.025%, most preferably in an amount of equal or less than 0.015%, that means from 0.001% to a maximum of 0.015% by weight based on the amount of the compound of the formula (I). It is commonly understood that release testing is performed without undue delay after the manufacturing of a batch of the product has been completed. Release testing is also formally required before the respective product batch can be marketed.

Furthermore, the pharmaceutical composition according to the invention - when investigated at the end of the product shelf life - contains 4-(4-amino-3-fluorophenoxy)pyridine-2-carboxylic acid methylamide (IUPAC: 4-(4-amino-3-fluorophenoxy)-N-methylpyridine-2-carboxamide) (AFP-PMA) in an amount of equal or less than 0.10%, that means from 0.001% to a maximum of 0.10%, preferably in an amount of equal or less than 0.08%, that means from 0.001% to a maximum of 0.08%, most preferably in an amount of equal or less than 0.05%, that means from 0.001% to a maximum of 0.05% by weight based on the amount of the compound of the formula (I).

Another aspect of the present invention is a film-coated pharmaceutical composition, preferably a tablet, comprising regorafenib and - when investigated for release testing - 4-(4-amino-3-fluorophenoxy)pyridine-2-carboxylic acid methylamide (IUPAC: 4-(4-amino-3-fluorophenoxy)-N-methylpyridine-2-carboxamide) (AFP-PMA) in an amount of equal or less than 0.050%, that means from 0.001% to a maximum of 0.050%, preferably in an amount of equal or less than 0.025%, that means from 0.001% to a maximum of 0,025%, most preferably in an amount of equal or less than 0.015%, that means from 0.001% to a maximum of 0.015% by weight based on the amount of regorafenib and at least one pharmaceutically acceptable excipient.

Preference is given to a tablet comprising regorafenib and - when investigated for release testing - 4-(4-amino-3-fluorophenoxy)pyridine-2-carboxylic acid methylamide (IUPAC: 4-(4-amino-3-fluorophenoxy)-N-methylpyridine-2-carboxamide) (AFP-PMA) in an amount of equal or less than 0.050%, that means from 0.001% to a maximum of 0.050%, preferably in an amount of equal or less than 0.025%, that means from 0.001% to a maximum of 0.025%, most preferably in an amount of equal or less than 0.015%, that means from 0.001% to a maximum of 0.015% by weight based on the amount of regorafenib and at least one pharmaceutically acceptable excipient wherein the tablet is coated by a coating comprising a polyvinyl alcohol based polymer in particular a partially hydrolysed polyvinyl alcohol polymer and optionally one or more further pharmaceutically acceptable excipients.

Still another aspect of the present invention is a film-coated pharmaceutical composition, preferably a tablet, comprising regorafenib and - when investigated at the end of the product shelf life - 4-(4-amino-3-fluorophenoxy)pyridine-2-carboxylic acid methylamide (IUPAC: 4-(4-amino-3-fluorophenoxy)-N-methylpyridine-2-carboxamide) (AFP-PMA) in an amount of equal or less than 0.10%, that means from 0.001% to a maximum of 0.10%, preferably in an amount of equal or less than 0.08%, that means from 0.001% to a maximum of 0.08%, most preferably in an amount of equal or less than 0.05%, that means from 0.001% to a maximum of 0.05% by weight based on the amount of regorafenib and at least one pharmaceutically acceptable excipient.

Preference is given to a tablet comprising regorafenib and - when investigated at the end of the product shelf life - 4-(4-amino-3-fluorophenoxy)pyridine-2-carboxylic acid methylamide (IUPAC: 4-(4-amino-3-fluorophenoxy)-N-methylpyridine-2-carboxamide) (AFP-PMA) in an amount of equal or less than 0.10%, that means from 0.001% to a maximum of 0.10%, preferably in an amount of equal or less than 0.08%, that means from 0.001% to a maximum of 0.08%, most preferably in an amount of equal or less than 0.05%, that means from 0.001% to a maximum of 0.05% by weight based on the amount of regorafenib and at least one pharmaceutically acceptable excipient wherein the tablet is coated by a coating comprising a polyvinyl alcohol based polymer in particular a partially hydrolysed polyvinyl alcohol polymer and optionally one or more further pharmaceutically acceptable excipients.

The pharmaceutical composition according to the invention can be packed into packaging systems like bottles or containers together with a desiccant like molecular sieve. Preferably the pharmaceutical composition according to the invention is packed in a bottle together with molecular sieve. More preferably the pharmaceutical composition which is a tablet comprising regorafenib optionally coated with a coating comprising polyvinyl alcohol is packed in a bottle together with molecular sieve. Most preferably the pharmaceutical composition which is a tablet comprising a solid dispersion comprising regorafenib coated with a coating comprising a polyvinyl alcohol based polymer is packed in a bottle together with molecular sieve.

In general, a molecular sieve is a material containing tiny pores of a precise and uniform size. The maximum size of the molecular or ionic species that can enter the pores of a molecular sieve material is controlled by the dimensions of the channels, e.g. 0.4 nm (= 4 A, Angstroem). Small molecules can enter the pores and are adsorbed while larger molecules are not. For instance, a water molecule is small enough and forced into the pores which act as a trap for the penetrating water molecules, which are retained within the pores.

A widely used molecular sieve material are aluminosilicate minerals, e.g. zeolites.

For the drug product a molecular sieve with a pore size of 0.3 nm (= 3 A, Angstroem) or 0.4 nm (= 4 A, Angstroem) is used because water molecules with a size of approximately 0.28 nm (= 2.8 A, Angstroem) are effectively trapped while larger molecules are not. Preference is given to molecular sieve with a pore size of or 0.4 nm (= 4 A, Angstroem). The used molecular sieve is highly effective with an adsorption capacity of at least 16% (w/w) at 25 °C even at high relative humidity of 80%.

Molecular sieves are commercially available like CAN TRI-SORB™ 4A from Sud-Chemie.

The pharmaceutical composition according to the invention is chemically stable for more than 18 months, preferably more than 24 months, most preferably more than 36 months during storage e.g. in climatic zones 1 to 2 , preferably in climatic zones 1 to 4b.

The pharmaceutical composition according to the invention is chemically stable and comprises 4-(4-amino-3-fluorophenoxy)pyridine-2-carboxylic acid methylamide (AFP-PMA) in an amount of or less than 0.100%, that means from 0.001% to a maximum of 0.100%, preferably in an amount of equal or less than 0.08%, that means from 0.001% to a maximum of 0.08%, most preferably in an amount of equal or less than 0.050%, that means from 0.001% to a maximum of 0.050% by weight based on the amount of regorafenib in the composition for at least 18 months, preferably at least 24 months, most preferably at least 36 months during storage e.g. in climatic zones 1 to 2, preferably in climatic zones 1 to 4b. Climatic zones are a well-known concept to define the storage conditions for long-term stability studies in order to determine the shelf-life of pharmaceutical products. For example, data obtained from storage at 25 °C and 60 % relative humidity are used to justify a shelf-life for climatic zones 1 to 2, whereas data obtained from storage at 40 °C and 75 % relative humidity are used to justify a shelf-life for climatic zones 1 to 4b.

The monthly increase rate of the amount of AFP-PMA in the pharmaceutical composition according to the invention during storage at 25°C / 60 % relative humidity is equal or less than 0.0015%, that means from 0.0001% to a maximum of 0.0015%, preferably equal or less than 0.001%, that means from 0.0001% to a maximum of 0.001% by weight based on the amount of regorafenib in the composition per month.

The monthly increase rate of the amount of AFP-PMA in the pharmaceutical composition according to the invention during storage at 30°C / 75 % relative humidity is equal or less than 0.0030%, that means from 0.0001% to a maximum of 0.0030%, preferably equal or less than 0.0025%, that means from 0.0001% to a maximum of 0.0025% by weight based on the amount of regorafenib in the composition per month.

Surprisingly the generation of side products preferably of 4-(4-amino-3-fluorophenoxy)pyridine-2-carboxylic acid methylamide during storage of the pharmaceutical composition according to the invention is less when the pharmaceutical composition according to the invention is co-packed with molecular sieve than co-packed with other desiccants like silica gel.

### Process for manufacturing (coating)

Pharmaceutical compositions according to the invention include but are not limited to granules, pellets, tablets, dragées, pills, melts or solid dispersions, preferably tablets, solid dispersions, pellets and granules, most preferably tablets, and may be prepared according to methods known to the art for the manufacture of pharmaceutical compositions which are for example described in WO 2006/026500.

The pharmaceutical compositions according to the invention, preferably a tablet comprising regorafenib, is coated according to methods known to the art like spraying the coating liquid in a pan or perforated drum coater onto the pharmaceutical composition provided that the outlet air temperature is equal to or below 42 °C, for example 20°C to 42°C, preferably equal to or below 40 °C, for example 30°C to 40°C, most preferably equal to or below 38 °C, for example 32°C to 38°C.

The solvent/vehicle used in the coating step for dissolving or dispersing the coating material is an aqueous solvent/vehicle, preferably water.

Another topic of the present invention is a pharmaceutical composition comprising regorafenib, a hydrate, solvate, metabolite or pharmaceutically acceptable salt of regorafenib, or a polymorph thereof, preferably regorafenib, wherein the pharmaceutical composition is coated by a coating wherein the coating is obtainable or obtained by a coating process wherein the outlet air temperature in the coating process is equal to or below 42 °C, for example 20°C to 42°C, preferably equal to or below 40 °C, for example 30°C to 40°C, most preferably equal to or below 38 °C, for example 32°C to 38°C. and the solvent/vehicle used in the coating step is an aqueous solvent/vehicle, preferably water.

The coating step with a coating material comprising a polyvinyl alcohol based polymer can be conducted by homogeneously dissolving or dispersing the coating material, for example Opadry™ II 85G35294 pink, Opadry™ II 85G25457 red, Opadry™ II 85G23665 orange, in the solvent/vehicle, for example water. Alternatively the coating material can be prepared from the individual components. The coating liquid then is sprayed on the pharmaceutical composition according to the invention, for example a tablet, in a perforated drum coater.

Surprisingly good coating results can be obtained at low coating temperatures, i.e. at outlet air temperatures equal to or below 42 °C, for example 20°C to 42°C, preferably equal to or below 40 °C, for example 30°C to 40°C, most preferably equal to or below 38 °C, for example 32°C to 38°C.

Surprisingly the amount of the 4-(4-amino-3-fluorophenoxy)pyridine-2-carboxylic acid methylamide (IUPAC: 4-(4-amino-3-fluorophenoxy)-N-methylpyridine-2-carboxamide) (AFP-PMA) from the uncoated to the final coated pharmaceutical composition according to the invention increases only by 0.0005 to 0.0030%, preferably by 0.0005 to 0.0020% by weight based on the amount of regorafenib.

Therefore another aspect of the present invention is a process for the manufacturing of a coated pharmaceutical composition, preferably a tablet, comprising regorafenib wherein the amount of the 4-(4-amino-3-fluorophenoxy)pyridine-2-carboxylic acid methylamide (IUPAC: 4-(4-amino-3-fluorophenoxy)-N-methylpyridine-2-carboxamide) (AFP-PMA) in the uncoated pharmaceutical composition to the final coated pharmaceutical composition increases only by 0.0005 to 0.0030%, preferably by 0.0005 to 0.0020% by weight based on the amount of regorafenib.

### Method for treatment:

The present invention also relates to a method for using the compound of the invention and compositions thereof, to treat mammalian hyper-proliferative disorders. This method comprises administering to a mammal in need thereof, including a human, an amount of a compound of the invention or composition thereof, which is effective to treat the disorder. Hyper-proliferative disorders include but are not limited to solid tumors, such as cancers of the breast, respiratory tract, brain, reproductive organs, digestive tract, urinary tract, eye, liver, skin, head and neck, thyroid, parathyroid and their distant metastases. Those disorders also include lymphomas, sarcomas, and leukemias.

Examples of breast cancer include, but are not limited to invasive ductal carcinoma, invasive lobular carcinoma, ductal carcinoma in situ, and lobular carcinoma in situ.

Examples of cancers of the respiratory tract include, but are not limited to small-cell and non-small-cell lung carcinoma, as well as bronchial adenoma and pleuropulmonary blastoma.

Examples of brain cancers include, but are not limited to brain stem and hypophtalmic glioma, cerebellar and cerebral astrocytoma, medulloblastoma, ependymoma, as well as neuroectodermal and pineal tumor.

Tumors of the male reproductive organs include, but are not limited to prostate and testicular cancer. Tumors of the female reproductive organs include, but are not limited to endometrial, cervical, ovarian, vaginal, and vulvar cancer, as well as sarcoma of the uterus.

Tumors of the digestive tract include, but are not limited to anal, colon, colorectal, esophageal, gallbladder, gastric, pancreatic, rectal, small intestine, and salivary gland cancers.

Preference is given to colorectal cancer.

Preference is also given to gastrointestinal stromal tumors (GIST).

Tumors of the urinary tract include, but are not limited to bladder, penile, kidney, renal pelvis, ureter, and urethral cancers.

Eye cancers include, but are not limited to intraocular melanoma and retinoblastoma.

Examples of liver cancers include, but are not limited to hepatocellular carcinoma (liver cell carcinomas with or without fibrolamellar variant), cholangiocarcinoma (intrahepatic bile duct carcinoma), and mixed hepatocellular cholangiocarcinoma.

Preference is given to hepatic cell cancer.

Skin cancers include, but are not limited to squamous cell carcinoma, Kaposi's sarcoma, malignant melanoma, Merkel cell skin cancer, and non-melanoma skin cancer.

Head-and-neck cancers include, but are not limited to laryngeal / hypopharyngeal / nasopharyngeal / oropharyngeal cancer, and lip and oral cavity cancer.

Lymphomas include, but are not limited to AIDS-related lymphoma, non-Hodgkin's lymphoma, cutaneous T-cell lymphoma, Hodgkin's disease, and lymphoma of the central nervous system.

Sarcomas include, but are not limited to sarcoma of the soft tissue, osteosarcoma, malignant fibrous histiocytoma, lymphosarcoma, and rhabdomyosarcoma.

Leukemias include, but are not limited to acute myeloid leukemia, acute lymphoblastic leukemia, chronic lymphocytic leukemia, chronic myelogenous leukemia, and hairy cell leukemia.

These disorders have been well characterized in humans, but also exist with a similar etiology in other mammals, and can be treated by administering pharmaceutical compositions of the present invention.

Based upon standard laboratory techniques known to evaluate compounds useful for the treatment of hyper-proliferative disorders, by standard toxicity tests and by standard pharmacological assays for the determination of treatment of the conditions identified above in mammals, and by comparison of these results with the results of known medicaments that are used to treat these conditions, the effective dosage of the compounds of this invention can readily be determined for treatment of each desired indication. The amount of the active ingredient to be administered in the treatment of one of these conditions can vary widely according to such considerations as the particular compound and dosage unit employed, the mode of administration, the period of treatment, the age and sex of the patient treated, and the nature and extent of the condition treated.

The present invention further provides the use of the compound of the invention for the preparation of a pharmaceutical compositions for the treatment of the aforesaid disorders.

### Combination with other pharmaceutical agents:

The compound of the invention can be administered as the sole pharmaceutical agent or in combination with one or more other pharmaceutical agents where the combination causes no unacceptable adverse effects. For example, the compound of the invention can be combined with known anti-hyper-proliferative or other indication agents, and the like, as well as with admixtures and combinations thereof.

Optional anti-hyper-proliferative agents which can be added to the compositions include but are not limited to compounds listed on the cancer chemotherapy drug regimens in the 11th Edition of the Merck Index, (1996), which is hereby incorporated by reference, such as asparaginase, bleomycin, carboplatin, cannustine, chlorambucil, cisplatin, colaspase, cyclophosphamide, cytarabine, dacarbazine, dactinomycin, daunorubicin, doxorubicin (adriamycine), epirubicin, etoposide, 5-fluorouracil, hexamethylmelamine, hydroxyurea, ifosfamide, irinotecan, leucovorin, lomustine, mechlorethamine, 6-mercaptopurine, mesna, methotrexate, mitomycin C, mitoxantrone, prednisolone, prednisone, procarbazine, raloxifen, streptozocin, tamoxifen, thioguanine, topotecan, vinblastine, vincristine, and vindesine.

Other anti-hyper-proliferative agents suitable for use with the compositions of the invention include but are not limited to those compounds acknowledged to be used in the treatment of neoplastic diseases in Goodman and Gilman's The Pharmacological Basis of Therapeutics (Ninth Edition), editor Molinoff et al., publ. by McGraw-Hill, pages 1225-1287, (1996), which is hereby incorporated by reference, such as aminoglutethimide, L-asparaginase, azathioprine, 5-azacytidine cladribine, busulfan, diethylstilbestrol, 2', 2'-difluorodeoxycytidine, docetaxel, erythrohydroxynonyladenine, ethinyl estradiol, 5-fluorodeoxyuridine, 5-fluorodeoxyuridine monophosphate, fludarabine phosphate, fluoxymesterone, flutamide, hydroxyprogesterone caproate, idarubicin, interferon, medroxyprogesterone acetate, megestrol acetate, melphalan, mitotane, paclitaxel, pentostatin, N-phosphonoacetyl-L-aspartate (PALA), plicamycin, semustine, teniposide, testosterone propionate, thiotepa, trimethylmelamine, uridine, and vinorelbine.

Other anti-hyper-proliferative agents suitable for use with the compositions of the invention include but are not limited to other anti-cancer agents such as epothilone and its derivatives, irinotecan, raloxifen and topotecan.

Generally, the use of the combinations of the present invention mentioned before will serve to:
(1) yield better efficacy in reducing the growth of a tumor or even eliminate the tumor as compared to administration of either agent alone,
(2) provide for the administration of lesser amounts of the administered chemotherapeutic agents,
(3) provide for a chemotherapeutic treatment that is well tolerated in the patient with fewer deleterious pharmacological complications than observed with single agent chemotherapies and certain other combined therapies,
(4) provide for treating a broader spectrum of different cancer types in mammals, especially humans,
(5) provide for a higher response rate among treated patients,
(6) provide for a longer survival time among treated patients compared to standard chemotherapy treatments,
(7) provide a longer time for tumor progression, and/or
(8) yield efficacy and tolerability results at least as good as those of the agents used alone, compared to known instances where other cancer agent combinations produce antagonistic effects.

"Combination" means for the purposes of the invention not only a dosage form which contains all the components (so-called fixed combinations), and combination packs containing the components separate from one another, but also components which are administered simultaneously or sequentially, as long as they are employed for the prophylaxis or treatment of the same disease.

It should be apparent to one of ordinary skill in the art that changes and modifications can be made to this invention without departing from the spirit or scope of the invention as it is set forth herein.

All publications, applications and patents cited above and below are incorporated herein by reference.

The weight data are, unless stated otherwise, percentages by weight and parts are parts by weight.

### Examples:

### Example 1: Coated tablet comprising regorafenib

### a) Solid dispersion

A solution of 0.415 kg of regorafenib monohydrate (corresponding to 0.40 kg regorafenib) and 1.60 kg of polyvinyl pyrrolidone (PVP 25) in a mixture of 4.80 kg acetone and 1.20 kg ethanol was prepared. Using a fluidized bed vacuum granulator this solution was sprayed onto a powder bed of 1.00 kg croscarmellose sodium and 1.00 kg microcrystalline cellulose at a temperature of 60 - 70°C.

### b) Tableting

The granulate of step a) was roller compacted and screened 3.15 mm and 1.0 mm. Subsequently the compacted granulate was blended with 0.54 kg croscarmellose sodium, 0.0240 kg colloidal anhydrous silica and 0.0360 kg magnesium stearate. This ready-to-press blend was compressed on a rotary tablet press into tablets containing 20 mg and 40 mg of regorafenib.

### c) Film coating

For coating of the 20 mg tablets 0.160 kg of Opadry™ II 85G35294 pink was homogeneously dispersed in 0.640 kg water. For coating of the 40 mg tablets 0.120 kg of Opadry™ II 85G35294 pink was homogeneously dispersed in 0.480 kg water. These coating suspensions were sprayed onto the 20 mg respectively 40 mg tablets of step b) in a perforated drum coater at an outlet air temperature of 35°C. The coating process resulted in evenly coated tablets with a smooth surface. Coating defects could not be observed.

Commercially available Opadry™ II 85G35294 pink contains polyvinyl alcohol (partially hydrolyzed) [44% by weight of the total mixture], polyethylenglycol (PEG 3350) [12.4% by weight of the total mixture], lecithin (soya), ferric oxides, titanium dioxide and talc.

**Table 2: Composition of tablets containing regorafenib**

| | Tablet A (20 mg) | Tablet B (40 mg) |
|---|---|---|
| | [mg/tablet] | [mg/tablet] |
| Regorafenib | 20.00 | 40.00 |
| Polyvinylpyrrolidone (PVP 25) | 80.00 | 160.00 |
| Croscarmellose sodium | 77.00 | 154.00 |
| Microcrystalline cellulose | 50.00 | 100.00 |
| Magnesium stearate | 1.80 | 3.60 |
| Silica colloidal anhydrous | 1.20 | 2.40 |
| Opadry II 85G35294 pink | 8.00 | 12.00 |
| Sum | 238.00 | 472.00 |
| Tablet format | round | oval |
| Dimensions of the tablet | diameter: 9 mm | Length: 16 mm, width: 7 mm |

The formulation of Example 1 has also been manufactured in different, i.e. larger scales. The ratio of ingredients and the operating principle of the equipment was the same.

### Example A: HPMC based coated tablet comprising regorafenib for comparison

Tablet cores equivalent to the uncoated tablets manufactured as described in Example 1 (a-b) were coated with a hydroxypropylmethyl cellulose (HMPC) based coating suspension (HPMC 15 cP 720 g, PEG 3350 24.0 g, Titanium dioxide 23.3 g, Ferric oxide red 0.72 g, water 1480 g) at an outlet air temperature of 60°C.

### Test results:

### Comparison between Example 1 and Example A

The degradation product 4-(4-amino-3-fluorophenoxy)pyridine-2-carboxylic acid methylamide (IUPAC: 4-(4-amino-3-fluorophenoxy)-N-methylpyridine-2-carboxamide) (AFP-PMA) was detected in the uncoated tablets in Example 1 after step b) in an amount of 0.0042% by weight based on the amount of regorafenib. After the final step c) in Example 1 AFP-PMA was detected in the coated tablet according to Example 1 in an amount of 0.0050% by weight based on the amount of regorafenib. The amount of AFP-PMA increased only by 0.0008%.

Analogous increase rate were observed when investigating tablets and coated tablets according to Example 1 manufactured in larger scale.

AFP-PMA was detected in the uncoated tablets used in comparative Example A in an amount of 0.0024% by weight based on the amount of regorafenib. After the coating (HPMC based coating) AFP-PMA was detected in the coated tablet according to Example A in an amount of 0.0078% by weight based on the amount of regorafenib. The amount of AFP-PMA increased by 0.0054%.

### Storage Stability of Example 1

Coated tablets according to Example 1 were packed in HDPE (high density polyethylene) bottles together with molecular sieve (CAN TRI-SORB™ 4A, 3g, Süd-Chemie) at a) 25°C and 60% relative humidity, and b) 30°C and 75% relative humidity.

Similarly, coated tablets according to Example 1 were packed in HDPE bottles together with silica gel (CAN SORB-IT™ 3g, Süd-Chemie) at a) 25°C and 60% relative humidity, and b) 30°C and 75% relative humidity.

The results of both stability studies are displayed in Table 3. A nearly linear increase in the amount of AFP-PMA was found in all studies. Therefore, the stability results are expressed as mean monthly increase rates determined on several batches over a period of up to 30 months.

The actual amount of AFP-PMA present in coated tablets containing regorafenib at the end of the shelf-life of the respective batch can be estimated by adding the respective monthly increments to the initial amount present in the coated tablets at the time of release testing. Likewise, the shelf-life of the product packed in bottles together with a desiccant in a climatic zone can be deduced.

**Table 3: Stability results of tablets containing regorafenib**

| | HDPE bottles containing tablets according to Example 1 with molecular sieve | HDPE bottles containing tablets according to Example 1 with silica |
|---|---|---|
| | Monthly increase rate [%/month] | Monthly increase rate [% / month] |
| Storage at 25 °C, 60% r.h. | 0.0008 | 0.0019 |
| Storage at 30 °C, 75% r.h. | 0.0020 | 0.0034 |

## Claims

1. A film-coated pharmaceutical composition comprising regorafenib and 4-(4-amino-3-fluorophenoxy)pyridine-2-carboxylic acid methylamide in an amount of equal or less than 0.050% by weight based on the amount of regorafenib and at least one pharmaceutically acceptable excipient.

2. The pharmaceutical composition of claim 1 wherein the amount of 4-(4-amino-3-fluorophenoxy)pyridine-2-carboxylic acid methylamide is equal or less than 0.025% by weight based on the amount of regorafenib.

3. The pharmaceutical composition of claim 1 wherein the amount of 4-(4-amino-3-fluorophenoxy)pyridine-2-carboxylic acid methylamide is equal or less than 0.015% by weight based on the amount of regorafenib.

4. The pharmaceutical composition of claim 1 wherein the amount of 4-(4-amino-3-fluorophenoxy)pyridine-2-carboxylic acid methylamide is from 0.001% to a maximum of 0.050% by weight based on the amount of regorafenib.

5. The pharmaceutical composition of claim 1 wherein the amount of 4-(4-amino-3-fluorophenoxy)pyridine-2-carboxylic acid methylamide is from 0.001% to a maximum of 0.025% by weight based on the amount of regorafenib.

6. The pharmaceutical composition of claim 1 wherein the amount of 4-(4-amino-3-fluorophenoxy)pyridine-2-carboxylic acid methylamide is from 0.001% to a maximum of 0.015% by weight based on the amount of regorafenib.

7. The pharmaceutical composition of any of claims 1 to 6 which is a tablet.

8. A pharmaceutical composition comprising regorafenib, a hydrate, solvate, metabolite or pharmaceutically acceptable salt of regorafenib, or a polymorph thereof and at least one pharmaceutically acceptable excipient wherein the pharmaceutical composition is coated by a coating comprising a polyvinyl alcohol based polymer and optionally one or more further pharmaceutically acceptable excipients.

9. The composition of any of claim 1 which is a tablet.

10. The composition of any of claims 1 to 2 is an immediate release tablet.

11. The composition of any of claims 1 to 3 wherein the polyvinyl alcohol based polymer is a hydrolysed polyvinyl alcohol polymer, a partially hydrolysed polyvinyl alcohol polymer, an esterified polyvinyl alcohol polymer, a co-polymer thereof with polyethylene glycol or a mixture of the thereof.

12. The composition of claim 4 wherein the polyvinyl alcohol based polymer is a partially hydrolysed polyvinyl alcohol polymer.

13. The composition of any of claims 1 to 5 wherein the polyvinyl alcohol based polymer is present in an amount of 30 to 70% by weight of the total coating.

14. The composition of any of claims 1 to 6 wherein the coating comprises polyethylene glycol, propylene glycol, sorbitol, glycerol, maltitol, xylitol, mannitol, erythritol, glycerol trioleate, tributyl citrate, triethyl citrate acetyl triethyl citrate, glyceryl triacetate, stearic acid, medium chain triglycerides or a mixture thereof as plasticizer.

15. The composition of claim 7 wherein the plasticizer is polyethylene glycol.

16. The composition of any of claims 7 or 8 wherein the plasticizer is in an amount of 5 to 30% by weight of the total coating.

17. The composition of any of claims 1 to 9 comprising a solid dispersion comprising regorafenib.

18. The composition of claim 10 comprising regorafenib in an amorphous state and a pharmaceutically acceptable matrix wherein the matrix comprises polyvinylpyrrolidone, vinylpyrrolidone/vinylacetate copolymer, polyalkylene glycol, hydroxyalkyl, hydroxyalkyl methyl cellulose, carboxymethyl cellulose, sodium carboxymethyl cellulose, ethyl cellulose, polymethacrylates, polyvinyl alcohol, polyvinyl acetate, vinyl alcohol/vinyl acetate copolymer, polyglycolized glycerides, xanthan gum, carrageenan, chitosan, chitin, polydextrin, dextrin, starch, proteins, sucrose, lactose, fructose, maltose, raffinose, sorbitol, lactitol, mannitol, maltitol, erythritol, inositol, trehalose, isomalt, inulin, maltodextrin, ß-cyclodextrin, hydroxypropyl-ß-cyclodextrin or sulfobutyl ether cyclodextrin or a mixture thereof.

19. The composition of any of claims 10 to 11 comprising regorafenib and the matrix agent in a weight ratio of 1:0.5 to 1:20.

20. The composition of any of claims 10 to 12 comprising regorafenib and polyvinylpyrrolidone, croscarmellose sodium and/or microcrystalline cellulose.

21. The composition of claim 13 comprising regorafenib and the sum of croscarmellose sodium and/or microcrystalline cellulose in a weight ratio of 1:0.5 to 1:20.

22. Film-coated pharmaceutical composition comprising regorafenib wherein the amount of 4-(4-amino-3-fluorophenoxy)pyridine-2-carboxylic acid methylamide is equal or less than 0.100 % by weight based on the amount of regorafenib.

23. Container containing molecular sieve and a pharmaceutical composition comprising regorafenib.

24. Pharmaceutical composition comprising regorafenib, a hydrate, solvate, metabolite or pharmaceutically acceptable salt of regorafenib, or a polymorph thereof, preferably regorafenib, wherein the pharmaceutical composition is coated by a coating wherein the coating is obtainable by a coating process wherein the outlet air temperature in the coating process is equal to or below 42 °C.

25. Process for the manufacture of a film-coated pharmaceutical composition comprising regorafenib wherein the coating liquid is sprayed onto the pharmaceutical composition in a pan or perforated drum coater and the outlet air temperature during the coating is equal to or below 42 °C.
